# EUROPEAN PATENT SPECIFICATION

(11) **EP 1 265 616 B1**
(45) Date of publication and mention of the grant of the patent: **17.05.2006**
(21) Application number: 01923331.1
(22) Date of filing: 16.03.2001
(51) Int. Cl.: A61K 31/57, A61P 5/30

(54) **HORMONE REPLACEMENT THERAPY USING A COMBINATION OF CONJUGATED ESTROGENS AND MEDROXYPROGESTERONE ACETATE**
HORMONERSATZTHERAPIE UNTER VERWENDUNG EINER KOMBINATIOIN VON KONJUGIERTEN OESTROGENEN UND MEDROXYPROGESTERON AZETAT
THERAPIE PAR REMPLACEMENT D'HORMONES UTILISANT UNE COMBINATION D'OESTROGENES CONJUGUES ET DE MEDROXYPROGESTERONE ACETATE

(30) Priority: 20.03.2000 US 190630 P; 14.02.2001 US 268607 P
(43) Date of publication of application: 18.12.2002
(73) Proprietor: Wyeth, Madison, New Jersey 07940-0874 (US)
(72) Inventor: PICKAR, James, H., Springfield, PA 19064 (US)
(74) Representative: Mannion, Sally Kim
(86) International application number: PCT/US2001/040302
(87) International publication number: WO 2001/070208

(56) References cited:
- EP-A- 0 309 263
- B.M.WOLFE, M.W.HUFF: "Effects of continuous low-dosage hormonal replacement therapy on lipoprotein metabolism in postmenopausal women" METABOLISM CLINICAL & EXPERIMENTAL, vol. 44, no. 3, 1995, pages 410-417, XP001053539
- H.SUMINO E.A.: "Serum level of vascular endothelial growth factor is decreased by hormone replacement therapy in postmenopausal women without hypercholesterolemia" ATHEROSCLEROSIS, vol. 148, no. 1, 2000, pages 189-195, XP001053480
- G.G.GIRAUD E.A.: "Effects of estrogen and progestin on aortic size and compliance in postmanopausal women" AMERICAN JOURNAL OF OBSTETRICS AND GYNECOLOGY, vol. 174, no. 6, 1996, pages 1708-1718, XP001053521

## Description

### BACKGROUND

This invention relates to methods and pharmaceutical compositions for providing hormone replacement therapy in perimenopausal, menopausal, and postmenopausal women through the continuous administration of combinations of conjugated estrogens and medroxyprogesterone acetate.

Menopause is generally defined as the last natural menstrual period and is characterized by the cessation of ovarian function, leading to the substantial diminution of circulating estrogen in the bloodstream. Menopause is usually identified, in retrospect, after 12 months of amenorrhea. It is not a sudden event, but is often preceded by a time of irregular menstrual cycles prior to eventual cessation of menses. Following the cessation of menstruation, the decline in endogenous estrogen concentrations is typically rapid. There is a decrease in serum estrogens from circulating levels ranging from 40-250 pg/mL of estradiol and 40-170 pg/mL of estrone during ovulatory cycles to less than 15 pg/mL of estradiol and 30 pg/mL of estrone in postmenopausal women.

As these estrogens decline during the time preceding (perimenopause) and following the menopause (postmenopause), various physiological changes may result, including vulvar and vaginal atrophy causing vaginal dryness, pruritus and dyspareunia, and vasomotor instability manifested as hot flushes. Other menopausal disturbances may include depression, insomnia, and nervousness. The long-term physiologic effects of postmenopausal estrogen deprivation may result in significant morbidity and mortality due to increase in the risk factors for cardiovascular disease and osteoporosis. Menopausal changes in blood lipid levels, a major component of the pathogenesis of coronary heart disease (CHD), may be precursors to increased incidence of ischemic heart disease, atherosclerosis, and other cardiovascular disease. A rapid decrease in bone mass of both cortical (spine) and trabecular (hip) bone can be seen immediately after the menopause, with a total bone mass loss of 1% to 5% per year, continuing for 10 to 15 years.

Estrogen replacement therapy (ERT) is beneficial for symptomatic relief of hot flushes and genital atrophy and for prevention of postmenopausal osteoporosis. ERT has been recognized as an advantageous treatment for relief of vasomotor symptoms. There is no acceptable alternative to estrogen treatment for the atrophic changes in the vagina; estrogen therapy increases the vaginal mucosa and decreases vaginal dryness. Long term ERT is the key to preventing osteoporosis because it decreases bone loss, reduces spine and hip fracture, and prevents loss of height. In addition, ERT has been shown to be effective in increasing high density lipoprotein-cholesterol (HDL-C) and in reducing low density lipoprotein cholesterol (LDL-C), affording possible protection against CHD. ERT also can provide antioxidant protection against free radical mediated disorders or disease states. Estrogens have also been reported to confer neuroprotection, and inhibit neurodegenerative disorders, such as Alzheimer's disease (see U.S. Patent 5,554,601, which is hereby incorporated by reference). The following table contains a list of estrogen preparations currently available.

**Estrogen replacement therapies available in the United States and/or Europe**

| Generic Name | Brand Name | Strength |
|---|---|---|
| Oral estrogens | | |
| Conjugated equine estrogens (natural) | Premarin | 0.3, 0.625, 0.9, 1.25, 2.5 mg |
| Conjugated estrogens (synthetic) | Cenestin | 0.625, 0.9 mg |
| Esterified estrogens (75-80% estrone sulfate 6-15% equilin sulfate derived from plant sterols) | Estratab | 0.3, 0.625, 1.25, 2.5 mg |
| Estropipate (Piperazine estrone sulfate) | Ogen Ortho-Est | 0.625, 1.25, 2.5 mg |
| Micronized estradiol | Estrace | 0.5, 1.0, 2.0 mg |
| Raloxifene (selective estrogen receptor modulator) | Evista | 60 mg |
| Esterified estrogens and methylestosterone | Estratest | 1.25 mg esterified estrogen and 2.5 mg methylestosterone |
| | Estratest HS | 0.625 mg esterified estrogen and 1.25 mg methylestosterone |

**Estrogen replacement therapies available in the United States and/or Europe (Continued)**

| Generic Name | | Brand Name | Strength |
|---|---|---|---|
| **Oral estrogens** | | | |
| Estradiol valerate | | Climaval | 1 mg, 2 mg |
| Estradiol | | Elleste Solo | 1 mg, 2 mg |
| Estradiol | | Estrofem | 2 mg |
| Estradiol | | Estrofem Forte | 4 mg |
| Piperazine esterone sulfate | | Harmogen | 1.5 mg |
| Combination: | Estrone | Hormonin | 1.4 mg |
| | Estradiol | | 0.6 mg |
| | Estriol | | 0.27 mg |
| Estradiol valerate | | Progynova | 1 mg, 2 mg |
| Estradiol | | Zumenon | 1 mg, 2 mg |
| | | | |
| **Transdermal estrogens** | | | |
| Estradiol | | Alora (twice weekly) | 0.025, 0.0375, 0.05, 0.075, |
| | | Climara (weekly) | 0.1 mg of estradiol released |
| | | Estraderm (2x weekly) | daily (dose options for various |
| | | Fem Patch (weekly) | products) |
| | | Vivelle (twice weekly) | |
| Estradiol | | Dermestril | 25, 50, 100 µg |
| Estradiol | | Estraderm | 25, 50, 100 µg |
| Estradiol | | Evorel (Systen) | 25, 50, 75, 100 µg |
| Estradiol | | Fematrix | 40, 80 µg |
| Estradiol | | Menorest | 25, 37.5, 50, 75 µg |
| | | Progynova TS | |
| Estradiol | | And TS Forte | 50, 100 µg |
| | | (Climara) | |
| | | | |
| **Vaginal estrogens** | | | |
| Conjugated equine estrogens | | Premarin vaginal | |
| Dienestrol | | cream | 0.625 mg/g |
| Estradiol | | Ortho dienestrol cream | 0.1 mg/g |
| Estropipate | | Estring | 7.5 µg |
| Micronized estradiol | | Ogen vaginal cream | 1.5 mg/g |
| | | Estrace vaginal cream | 1.0 mg/g |

To minimize the occurrence of estrogen-related side effects and to maximize the benefit-risk ratio, the lowest dose effective in relief of symptoms and prevention of osteoporosis should be used. Although ERT reduces the relative risk (RR) for ischemic heart disease (RR, 0.50) and osteoporosis (RR, 0.40), the relative risk of endometrial cancer for postmenopausal women with a uterus may be increased. There are extensive clinical data showing that the relative risk of endometrial cancer can be reduced by the addition of a progestin, either sequentially or continuously. The addition of a progestin to estrogen therapy prevents estrogen-induced endometrial proliferation. Continuous combined hormone replacement therapy (HRT), with appropriate doses of daily estrogen and progestin, has been shown to be effective in relieving vaginal atrophy and vasomotor symptoms, preventing postmenopausal osteoporosis, and reducing the risk of endometrial cancer by prevention of endometrial hyperplasia. The following table contains a list of some currently available oral combination HRT products.

**Oral Combination HRT Products**

| Brand Name | Estrogen/Progestin | Strengths |
|---|---|---|
| Activelle | Estradiol | 1 mg |
| | Norethisterone acetate (NETA) | 0.5 mg |
| Climagest | Estradiol valerate (Climaval) | 1 or 2 mg |
| | Norethisterone (NET) | 1 mg days 17-28 |
| Cyclo Progynova | Estradiol valerate | 1 or 2 mg, days 1-21 |
| | Levonorgestrel | 250 or 500 µg, days 2-21 |
| Elleste Duet | Estradiol | 1 or 2 mg |
| | Norethisterone acetate | 1 mg days 17-28 |
| Femoston | Estradiol | 1 or 2 mg |
| | Dydrogesterone | 10 or 20 mg |
| Kliogest | Estradiol | 2 mg |
| | Norethisterone acetate | 1 mg |
| Improvera | Piperazine estrone sulfate | 1.5 mg |
| | Medroxyprogesterone acetate (MPA) | 10 mg, days 17-28 |
| Nuvelle | Estradiol valerate (Progynova) | 2 mg |
| | Levonorgestrel | 75 µg, days 17-28 |
| Premphase | Conjugated estrogens | 0.625 mg |
| | MPA | 5.0mg |
| Prempro | Conjugated estrogens | 0.625 mg |
| | MPA | 2.5 or 5.0 mg |
| Trisequens | Estradiol | 2 or 4 mg, days 1-22 |
| And | Norethisterone | 1 mg, days 23-28 |
| Trisequens Forte | | 1 mg, days 13-22 |
| Ortho-Prefest | Estradiol | 1.0 mg, days 1-6 |
| | Nogestimate | 0.09 mg, days 4-6 |
| Femhrt 1/5 | Ethinyl estradiol | 1.0 mg |
| | Norethindrone acetate | 5 µg |

Since it is possible that progestins ameliorate of the favorable estrogen effects on lipids and may potentially impair of glucose tolerance, it is desirable, and an objective to find the lowest dose estrogen plus progestin HRT product, which also minimizes or eliminates endometrial hyperplasia. In addition, a major factor affecting a woman's decision to start and to continue taking HRT is vaginal bleeding, and many women would prefer a bleed-free product. Therefore, another objective is to provide the lowest effective dose which provides an acceptable bleeding pattern. Doses as low as NETA 0.5 mg, NET 0.35 mg, medroxyprogesterone acetate (MPA) 2.5 mg, levonorgesterel 0.25 mg, and dydrogesterone 5 mg have been used previously in continuous uninterrupted HRT regimens.

Thus, according to the present invention there is provided a pharmaceutical pack comprising daily dosage units comprising a combination of conjugated estrogens and a daily dosage of about 1.5 mg of medroxyprogesterone acetate, and further provided is the use of conjugated estrogens and medroxyprogesterone acetate in the manufacture of such pharmaceutical packs for the treatment or inhibition of menopausal or postmenopausal disorders in a perimenopausal, menopausal, or postmenopausal woman in need thereof.

### BRIEF DESCRIPTION OF THE DRAWINGS

FIG. 1 shows the mean number of hot flushes per day in patients receiving PREMARIN plus MPA combinations or placebo.
FIG. 2 shows the mean severity of hot flushes in patients receiving PREMARIN plus MPA combinations or placebo.
FIG. 3 shows the percentage of patients with amenorrhea in patients receiving PREMARIN plus MPA combinations or placebo.

### DESCRIPTION OF THE INVENTION

The purpose of this invention is to provide the significant benefits of a commercially successful HRT product, such as PREMPRO (0.625 mg conjugated equine estrogens, USP plus 2.5 mg MPA), while lowering the dosage of MPA below that which has previously been demonstrated to be effective, and preferably also lowering the dosage of the conjugated estrogens. This invention provides a method of treating or inhibiting menopausal or postmenopausal disorders in a perimenopausal, menopausal, or postmenopausal woman in need thereof, which comprises providing to said woman, continuously and uninterruptedly over the treatment period, a combination of conjugated estrogens (natural or synthetic) and a daily dosage of about 1.5 mg MPA. The dosage is preferably provided as a pharmaceutical composition for use in treating menopausal or postmenopausal disorders which comprises a combination of conjugated estrogens and a dosage of about 1.5 mg MPA. This invention further provides a pharmaceutical pack containing the daily dosage units of conjugated estrogen and MPA for continuous daily administration.

Conjugated estrogens refer to estrogenic steroidal substances in which one or more functional groups (typically hydroxyl groups) on the steroid exists as a conjugate (typically a sulfate or glucuronide). The conjugated estrogens may be a single conjugated estrogen, or may consist of mixtures of various conjugated estrogens. Numerous conjugated estrogens are described in the literature or are commercially available that are capable of being formulated for use in this invention either as a unitary estrogen, or may be mixed together with other synthetic and/or natural estrogens.

Conjugated estrogens may also contain other steroidal or non-steroidal compounds, which may, or may not, contribute to the overall biological effect. Such compounds include, but are not limited to, unconjugated estrogens, androstanes, and pregnanes. Preferred conjugated estrogens for use in this invention are PREMARIN (conjugated equine estrogens, USP) and CENESTIN (synthetic conjugated estrogens, A).

PREMARIN (conjugated estrogens tablets, USP) for oral administration contains a mixture of estrogens obtained exclusively from natural sources, occurring as the sodium salts of water-soluble estrogen sulfates blended to represent the average composition of material derived from pregnant mares' urine. It is a mixture of sodium estrone sulfate and sodium equilin sulfate, and at least the following 8 concomitant components, also as sodium sulfate conjugates: 17α-dihydroequilin, 17a-estradiol, Δ8,9-dehydroestrone, 17β-dihydroequilin, 17β-estradiol, equilenin, 17α-dihydroequilenin, and 17β-dihydroequilenin. The make up of PREMARIN of PREMARIN is currently being analyzed, and other components are in the process of being identified and characterized. PREMARIN is indicated in the treatment of moderate to severe vasomotor symptoms associated with the menopause; treatment of vulvar and vaginal atrophy; and prevention of osteoporosis, as well as other indications approved for estrogen products.

CENESTIN (synthetic conjugated estrogens, A) tablets for oral administration contain a blend of 9 synthetic estrogenic substances: sodium estrone sulfate, sodium 17α-dihydroequilin sulfate, sodium 17α-estradiol sulfate, sodium equilenin sulfate, sodium 17α-dihydroequilenin sulfate, sodium equilin sulfate, sodium 17β-dihydroequilin sulfate, sodium 17β-estradiol sulfate, sodium 17α-dihydroequilenin sulfate. CENESTIN is indicated in the treatment of moderate to severe vasomotor symptoms associated with the menopause.

PREMARIN, CENESTIN, and medroxyprogesterone acetate are all available from commercial sources (Wyeth-Ayerst - PREMARIN and medroxyprogesterone acetate; Duramed - CENESTIN). The dosage of MPA is about 1.5 mg per day. It is preferred that the conjugated estrogen constituent is PREMARIN. It is preferred that the dosage of PREMARIN is about 0.625 mg per day or less, and is more preferred that the dosage of PREMARIN is either about 0.45 mg per day or about 0.30 mg per day.

As used in accordance with this invention, the term "menopausal or postmenopausal disorder" refers to conditions, disorders, or disease states that are at least partially caused by the decreased estrogen production occurring during the perimenopausal, menopausal, or post-menopausal stages of a woman's life. Such disorders typically include, but are not limited to, one or more of :
- vasomotor instability, vasomotor symptoms such as hot flushes;
- bone demineralization causing reduced bone mineral density, increased risk of developing osteoporosis;
- vaginal or vulvar atrophy; atrophic vaginitis; vaginal dryness; pruritus; dyspareunia; dysuria;
- frequent urination; urinary incontinence; urinary tract infections,
- raised cholesterol, triglycerides, Lp(a), or LDL levels; hypercholesteremia; hyperlipidemia;
- cardiovascular disease; atherosclerosis; peripheral vascular disease; restenosis, vasospasm; vascular wall damage from cellular events leading toward immune mediated vascular damage;
- free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures;
- dementias, neurodegenerative disorders, and Alzheimer's disease.

As used herein, menopausal also includes conditions of decreased estrogen production that may be surgically, chemically, or be caused by a disease state which leads to premature diminution or cessation of ovarian function.

The term "daily" means that the dosage is to be administered at least once a day. The frequency of administration is preferred to be once a day, but may be more than once a day, provided that any specified daily dosage is not exceeded.

The term "combination" of conjugated estrogens and MPA means that the daily dosage of each of the components of the combination is administered during the treatment day. The components of the combination are preferably administered at the same time; either as a unitary dosage form containing both components, or as separate dosage units; the components of the combination can be administered at different times during the day, provided that the desired daily dosage is achieved.

The term "continuous and uninterrupted" means that there is no break in the treatment regimen, during the treatment period. Thus, "continuous, uninterrupted administration" of a combination, means that the combination is administered at least once daily during the entire treatment period. It is expected that the treatment period for the combination of conjugated estrogens and MPA will be for at least 28 days, preferably 30 days and preferably for long term treatment, possibly an indefinite period, as one of the primary reasons for administering combinations of conjugated estrogens and MPA is to treat or inhibit menopausal or postmenopausal disorders. It is preferred that the treatment is provided for at least 120 days, preferably 180 days, and most preferably as an indefinite treatment. Pharmaceutical packs suitably contain sufficient daily dosage units appropriate to the treatment period or in amounts which facilitate the patient's compliance with the regimen. For example, the pharmaceutical pack may contain from 28 to 180 daily dosage units, for example from 30 to 120 daily dosage units. Pharmacaceutical packs containing large numbers of dosage units may be further sub-packaged, for example, in 28 day periods, or 30 day periods. Treatment periods also may vary depending on the symptoms to be treated. For example, for the treatment of vasomotor symptoms, it is preferred that the treatment may last from one month to several years, depending on the severity and duration of the symptoms. Physician evaluation along with patient interaction will assist the determination of the duration of treatment. For the treatment or inhibition of osteoporosis, it is preferred that the treatment period could last from six months to a number of years, or indefinitely.

This invention, also covers short term treatments or treatments of a finite term, that may be less than the 28 or 30 day preferred treatment period. It is anticipated that a patient may miss, or forget to take, one or a few dosages during the course of a treatment regimen, however, such patient is still considered to be receiving continuous, uninterrupted administration.

The term "fixed daily dosage" means that the same dosage amount is given every day during the treatment period. It is preferred that the MPA is given in a fixed daily dosage of about 1.5 mg, with an appropriate dose of conjugated estrogens, preferably equivalent to about 0.45 mg or about 0.30 mg PREMARIN. One aspect of this invention also covers situations in which a fixed daily dosage of the conjugated estrogens plus MPA combination is not given every day during the treatment period. For example, the dosage of a patient may need to be adjusted (either up or down), to achieve the desired effect during the middle of a treatment period.

The term "providing," with respect to providing a dosage of one or both of the components of this invention, means either directly administering such a component of this invention, or administering a prodrug, derivative, or analog which will form the equivalent amount of the component within the body.

The daily dosage unit is preferably provided in a form for oral administration containing both the conjugated estrogens and the MPA.

The ability of the conjugated estrogens plus MPA combinations of this invention to treat or inhibit menopausal or postmenopausal disorders was confirmed in a double blind clinical study of postmenopausal women using combinations of PREMARIN plus MPA, or placebo. In this study, patients received continuous and uninterrupted treatment for 13 cycles (1 year). The relief of vasomotor symptoms, prevention of endometrial hyperplasia, and effects on lipids, vaginal bleeding were measured throughout the study. Additionally, the effect on bone mineral density was evaluated in patients who received continuous and uninterrupted treatment for up to 26 cycles (2 years).

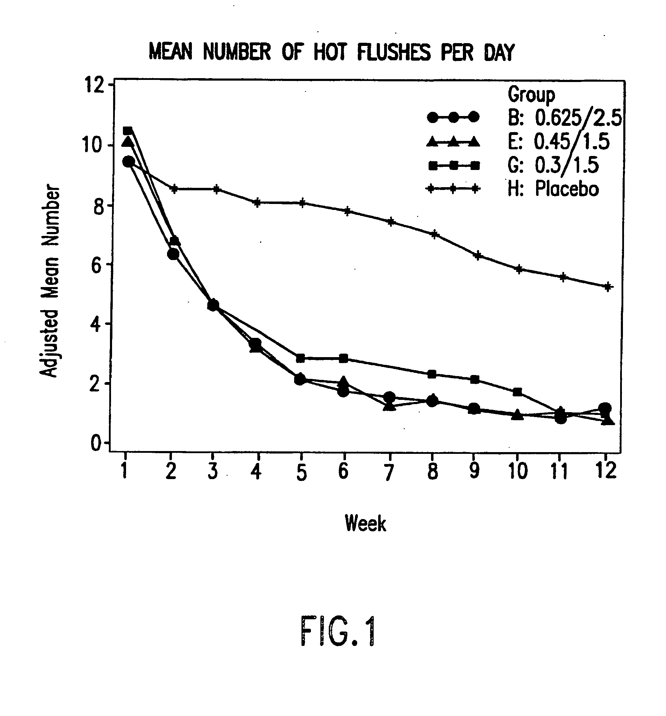

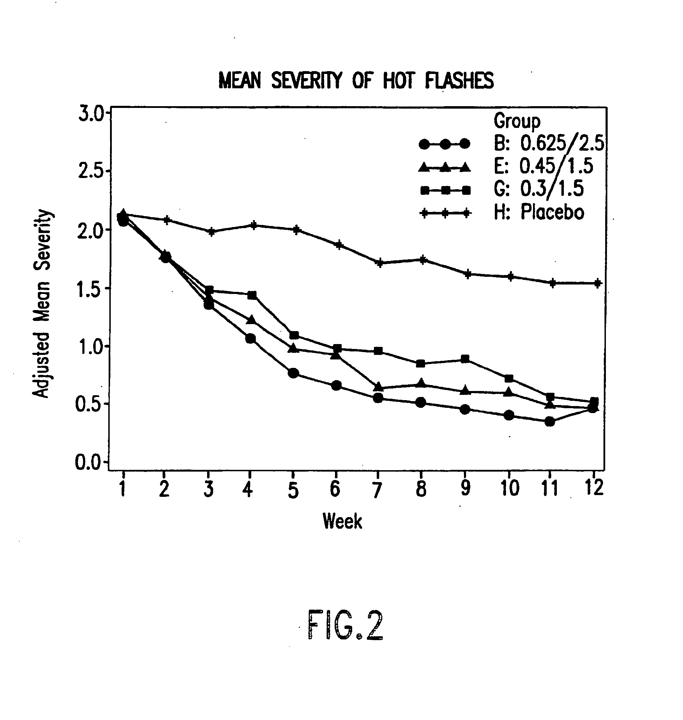

Vasomotor instability is a menopausal or postmenopausal disorder which is often manifested as hot flushes. In the clinical study described above, relief of vasomotor symptoms was analyzed in a subset of patients who experienced at least an average of 7-8 moderate-to-severe hot flushes per day during the 7-day period just prior to the initiation of treatment in this study. The results obtained are summarized in the tables below. The first table shows the mean number of flushes, and the second table shows the mean daily severity of the flushes. These results are also shown in FIGS. 1 and 2.

**Mean Number (± S.E.) of Hot Flushes Per Day**

| **Week** | **Treatment Group** | | | |
|---|---|---|---|---|
| | **Placebo** | **0.625 P + 2.5 M*** | **0.45 P + 1.5 M** | **0.3 P + 1.5 M** |
| 1 | 9.41 ± 0.81 | 9.50 ± 0.73 | 9.99 ± 0.79 | 10.60 ±0.76 |
| 2 | 8.55 ± 0.80 | 6.38 ± 0.72 | 6.98 ± 0.80 | 6.88 ± 0.74 |
| 3 | 8.51 ± 0.76 | 4.47 ± 0.69 | 4.47 ± 0.76 | 4.62±0.71 |
| 4 | 8.09 ± 0.72 | 3.38 ± 0.66 | 3.23 ± 0.72 | 3.84 ± 0.67 |
| 8 | 7.10 ± 0.65 | 1.55 ± 0.61 | 1.49 ± 0.65 | 2.41 ± 0.60 |
| 12 | 5.36 ± 0.55 | 1.16 ± 0.49 | 0.94 ± 0.53 | 1.13 ± 0.50 |

| | | | | |
|---|---|---|---|---|
| *Daily dosages of P (PREMARIN) and M (MPA). | | | | |

**Mean Severity (± S.E.) of Hot Flushes**

| **Week** | **Treatment Group** | | | |
|---|---|---|---|---|
| | **Placebo** | **0.625 P + 2.5 M*** | **0.45 P + 1.5 M** | **0.3 P + 1.5 M** |
| 1 | 2.10 ± 0.10 | 2.08 ± 0.09 | 2.14 ± 0.10 | 2.07 ± 0.10 |
| 2 | 2.06 ± 0.13 | 1.75 ± 0.12 | 1.78 ± 0.13 | 1.73 ± 0.12 |
| 3 | 1.97 ± 0.14 | 1.36 ± 0.13 | 1.42 ± 0.15 | 1.48 ± 0.14 |
| 4 | 2.03 ± 0.15 | 1.07 ± 0.14 | 1.21 ± 0.15 | 1.45 ± 0.14 |
| 8 | 1.75 ± 0.16 | 0.54 ± 0.14 | 0.70 ± 0.16 | 0.87 ± 0.14 |
| 12 | 1.57 ± 0.16 | 0.51 ± 0.14 | 0.51 ± 0.16 | 0.56 ± 0.14 |

| | | | | |
|---|---|---|---|---|
| *Daily dosages of P (PREMARIN) and M (MPA). | | | | |

As shown in both tables and Figures, all dosages of PREMARIN plus MPA reduced the number and severity of hot flushes experienced by the women in this clinical study compared with women taking placebo. All differences from placebo were significant (p < 0.05) by weeks 3-4. It was unexpected, however, that the lower dosages of PREMARIN (0.45 and 0.3 mg) and MPA (1.5 mg), would rapidly reduce the number and severity of hot flushes to the same extent as the higher dose combination containing 0.625 mg PREMARIN plus 2.5 mg MPA.

Vaginal atrophy is a common menopausal or postmenopausal disorder leading to vaginal dryness, pruritus, and dyspareunia. Vaginal atrophy results from a sloughing of vaginal epithelial cells which are not replaced, leading to a thinning of the vaginal lining. The effects of the lower dose conjugated estrogen plus MPA regimens on vaginal atrophy were evaluated by comparing the vaginal maturation index of superficial cells at baseline, and after cycles 6 and 13 of treatment. The vaginal maturation index is a measure of the number of superficial vaginal epithelial cells. An increase (positive number) in the vaginal maturation index indicates a reversal (successful treatment) of vaginal atrophy. The following table summarizes the results obtained.

**Vaginal Maturation Index for Superficial Cells - Median Change from Baseline**

| Treatment Group | Cycle 6 | Cycle 13 |
|---|---|---|
| 0.625 P + 2.5 M* | 10 | 10 |
| 0.45 P + 1.5 M | 10 | 10 |
| 0.3 P + 1.5 M | 10 | 10 |
| Placebo | 0 | 0 |

| | | |
|---|---|---|
| *Daily dosages of P (PREMARIN) and M (MPA). | | |

These data show that all the evaluated dosages of conjugated estrogens plus MPA provided significant (p < 0.001) improvement in the vaginal maturation index versus placebo, demonstrating their ability to successfully treat or inhibit vaginal atrophy. It is notable that the lower dosages of conjugated estrogens plus MPA were as equally as effective as the 0.625 mg PREMARIN plus 2.5 mg MPA dosage in facilitating the growth of the vaginal superficial cells.

As HRT using estrogens alone has been shown to increase the relative risk of endometrial hyperplasia in postmenopausal women with a uterus, the incidence of endometrial hyperplasia was evaluated in the clinical study for patients treated with PREMARIN plus MPA treated groups and placebo. Two independent pathologists evaluated endometrial biopsies in a blinded fashion. A patient was considered to have endometrial hyperplasia if both of the primary pathologists agreed on the diagnosis. If they disagreed, a third pathologist was consulted, and the diagnosis of hyperplasia was based on the diagnosis of the majority. The following table summarizes the results obtained after 13 cycles of treatment.

**Percent of Patients Developing Endometrial Hyperplasia**

| Treatment Group (Dosage)* | No. Hyperplasias | No. Patients/Group | Hyperplasia Rate (%) |
|---|---|---|---|
| Placebo | 0 | 261 | 0.00 |
| PREMARIN/MPA (0.625/2.5) | 0 | 278 | 0.00 |
| PREMARIN/MPA (0.45/1.5) | 1 | 272 | 0.37 |
| PREMARIN/MPA (0.3/1.5) | 1 | 272 | 0.37 |

| | | | |
|---|---|---|---|
| * All dosages are in mg/day | | | |

These results showed that that the use of conjugated equine estrogens/MPA at a dosage of 0.625/2.5 mg/day effectively prevented the development of endometrial hyperplasia. The results also show the unexpected result that lowering the dosage of MPA to 1.5 mg/day in PREMARIN plus MPA combinations, also continued to effectively inhibit the development of endometrial hyperplasia. The difference between the results obtained in the 1.5 mg MPA treatment groups and 2.5 mg MPA treatment groups was not statistically significant.

In providing an HRT regimen that will be acceptable to menopausal, and particularly postmenopausal women, it is highly desirable that the regimen produce a high rate of amenorrhea, as most of these women prefer a product which does not cause spotting or breakthrough bleeding. The following table shows the percent of women experiencing amenorrheic cycles at during cycles 1, 3, 6, 9, and 13.

**Percent Cycles of Amenorrhea**

| **Cycle** | **Treatment Group** | | | |
|---|---|---|---|---|
| | **Placebo** | **0.625 P + 2.5 M** | **0.45 P + 1.5 M** | **0.3 P + 1.5 M** |
| 1 | 91.1 | 51.3 | 71.1 | 75.2 |
| 3 | 96.4 | 54.9 | 70.3 | 80.4 |
| 6 | 91.8 | 68.4 | 72.8 | 85.1 |
| 9 | 93.6 | 70.8 | 80.5 | 86.6 |
| 13 | 95.2 | 77.4 | 79.8 | 88.4 |

| | | | | |
|---|---|---|---|---|
| *Daily dosages of P (PREMARIN) and M (MPA). | | | | |

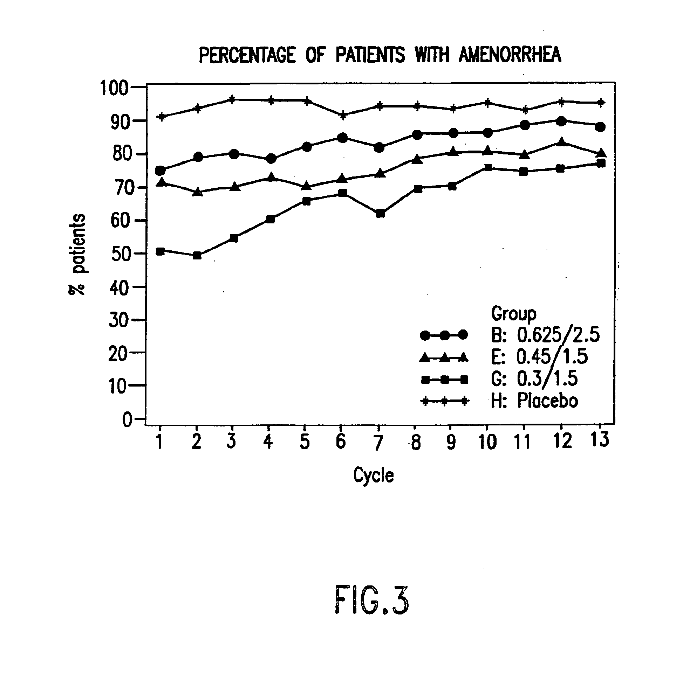

The results show that greater than 90 percent of women receiving placebo were amenorrheic throughout the study. While the percent of amenorrheic women receiving daily dosages of 0.625 mg PREMARIN plus 2.5 mg MPA is satisfactory, as measured by the commercial success of PREMPRO (0.625 mg conjugated equine estrogens plus 2.5 mg MPA), lowering the dosages of PREMARIN to either 0.45 mg or 0.3 mg and MPA to 1.5 mg, produced an equal, if not significantly better (0.3 mg PREMARIN plus 1.5 mg MPA) percent of women achieving amenorrhea, while still maintaining the other benefits of HRT. Additionally, as shown in the above table, and in FIG. 3, a higher percentage of amenorrhea was achieved more rapidly with the lower dose combinations.

It is well known that the addition of progestins to ERT regimens may ameliorate some of the beneficial cardioprotective effects conferred by the estrogen, or even produce deleterious effects on the lipid levels. In this study total cholesterol (TC), HDL, HDL₂, and LDL levels were measured. There was a general dose-response trend between treatment groups, that showed more favorable lipid profiles with higher doses of PREMARIN and lower doses of MPA. Patients receiving 0.625 mg PREMARIN + 2.5 mg MPA had slight reductions in TC, significant increases in HDL and HDL₂, and significant decreases in levels of LDL. The 0.45 mg PREMARIN plus 1.5 mg MPA dosage produced a similar favorable profile (but of less magnitude) to 0.625 mg PREMARIN + 2.5 mg MPA treated women. Women treated with 0.3 mg PREMARIN plus 1.5 mg MPA had a less favorable lipid profile (TC, HDL, HDL₂ and LDL), than women treated, with 0.625 mg PREMARIN plus 2.5 mg MPA, however, this profile was still better than those receiving placebo.
During the study, adverse events were recorded and analyzed. Treatment emergent adverse events were consistent with those seen with hormone therapy. With the exception of breast pain, the side effect profile was comparable between the PREMARIN plus MPA treatment groups. Women receiving the daily dosage of 0.3 mg PREMARIN plus 1.5 mg MPA experienced significantly less breast pain than the women taking 0.625 mg PREMARIN plus 2.5 mg MPA.

In summary the results of the clinical study demonstrated that conjugated estrogen HRT regimens containing dosages of 1.5 mg/day of MPA were equally effective in treating menopausal or postmenopausal disorders as the regimens containing the higher dose of 2.5 mg MPA (0.625 mg PREMARIN plus 2.5 mg MPA, in particular). Higher rates of amenorrhea were also achieved more rapidly.. Additionally, less breast tenderness was observed in women taking 0.3 mg PREMARIN plus 1.5 mg MPA, than in women taking the commercially available 0.625 mg PREMARIN plus 2.5 mg MPA combination.

It is well known that a rapid decrease in bone mass of both cortical (spine) and trabecular (hip) bone can be seen immediately after the menopause, with a total bone mass loss of 1% to 5% per year, continuing for 10 to 15 years. In the clinical study described above, bone mineral density (BMD) was determined using dual energy x-ray absorptiometry (DEXA) measurements of the lumbar spine (L2-L4), femoral neck, trochanter, and total body. BMD measurements were made at least twice pre-study (7-14 days apart but not to exceed 3 weeks), during cycles 6, 13, 19, and twice during cycle 26 (7-14 days apart but not to exceed 3 weeks). The final visit results (cycle 26) are summarized in the table below.

**Percent BMD Change ( ± S.E.) From Baseline at Final Visit**

| **Treatment Group** | **Lumbar Spine** | **Femoral Neck** | **Trochanter** | **Total Body** |
|---|---|---|---|---|
| Placebo | -2.63 ± 0.37⁺ | -1.97 ± 0.46⁺ | 0.82 ± 0.58 | -1.56 ± 0.17⁺ |
| 0.625 P + 2.5 M* | 3.77 ± 0.37^{+,#} | 1.67 ± 0.46^{+,#} | 4.05 ± 0.59^{+,#} | 0.96 ± 0.18^{+,#} |
| 0.45 P + 1.5 M | 2.45 ± 0.37^{+,#} | 1.43 ± 0.47^{++,#} | 3.60 ± 0.59^{+,#} | 0.56 ± 0.18^{+,#} |
| 0.3 P + 1.5 M | 1.77 ± 0.36^{+,#} | 1.51 ± 0.44^{+,#} | 4.66 ± 0.56^{+,#} | 0.55 ± 0.17^{+,#} |

| | | | | |
|---|---|---|---|---|
| * Daily dosages of P (PREMARIN) and M (MPA). | | | | |
| ⁺ Statistically significant (p < 0.001) change from baseline. | | | | |
| ⁺⁺ Statistically significant (p = 0.004) change from baseline. | | | | |
| ^{#} Statistically significant (p < 0.001) difference from placebo. | | | | |

The results showed that all dosages of PREMARIN plus MPA significantly increased the BMD versus baseline and placebo in the lumbar spine, femoral neck, trochanter, and total body demonstrating that combinations of conjugated estrogens plus MPA inhibited or retarded bone demineralization. These data also show that combinations of conjugated estrogens plus MPA actually increased the bone mineral density relative to pre-study baseline levels, and also relative to patients receiving placebo.

Based on the results observed in the clinical study described above, it has been found that the continuous and uninterrupted administration of a combination conjugated estrogens plus a dosage of about mg per of medroxyprogesterone acetate is useful in treating or inhibiting menopausal or postmenopausal disorders in perimenopausal, menopausal, or postmenopausal women (most particularly the latter). More particularly, the combinations described herein are useful in treating or inhibiting vaginal or vulvar atrophy; atrophic vaginitis; vaginal dryness; pruritus; dyspareunia; dysuria; frequent urination; urinary incontinence; urinary tract infections; vasomotor symptoms, including hot flushes, myalgia, arthralgia, insomnia, irritability, and the like; inhibiting or retarding bone demineralization; increasing bone mineral density; and treating or inhibiting osteoporosis.

The combinations of this invention also exert a cardioprotective effect in perimenopausal, menopausal, and postmenopausal women, and are therefore useful in lowering cholesterol, triglycerides, Lp(a), and LDL levels; inhibiting or treating hypercholesteremia; hyperlipidemia; cardiovascular disease; atherosclerosis; peripheral vascular disease; restenosis, and vasospasm; and inhibiting vascular wall damage from cellular events leading toward immune mediated vascular damage.

The combinations of this invention are antioxidants, and are therefore useful in inhibiting disorders or disease states which involve free radicals. More particularly, the combinations of this invention are useful in treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures.

The combinations of this invention are useful in treating or inhibiting dementias, neurodegenerative disorders, and Alzheimer's disease; providing neuroprotection or cognition enhancement.

The conjugated estrogens and medroxyprogesterone acetate described in this invention can be either formulated as separate tablets or as a unitary combination tablet.

Either of the components or the combination may be formulated neat or may be combined with one or more pharmaceutically acceptable carriers for administration. For example, solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the standpoint of ease of preparation and administration are solid compositions, particularly tablets and hard-filled or liquid-filled capsules. Oral administration of the compounds is preferred.

Conjugated estrogens and MPA can be formulated in a number of ways to provide a single combination dosage form. Conjugated estrogens can be incorporated within the core of a compressed tablet and the progestin can be placed in an overcoating consisting of a compressed, film or sugar coat, as described in U.S. Patent 5,547,948, which is hereby incorporated by reference. The tablets described in U.S. Patent 5,547,948 are suitable for formulation of the conjugated estrogens and MPA described in this invention as a unitary tablet. U.S. Patent 5,908,638, which is hereby incorporated by reference, also describes combination tablets which are suitable for formulation of the conjugated estrogens and MPA described in this invention as a unitary tablet.

Conjugated estrogens may be formulated in a core containing the conjugated estrogens, and several components including alcohol, hydroxypropyl methyl cellulose, lactose monohydrate, magnesium stearate, and starch. The core can be covered with a coating made from components such as ethylcellulose, and triethyl citrate.

Both components can be incorporated in the compressed tablet core or in a tablet coating formulated to maintain drug stability and provide adequate oral bioavailability. For example, the progestin can be micronized.

Conjugated estrogens can be incorporated in granules, spheroids or other multiparticulate forms, and, if necessary, coated to provide adequate stability. These multiparticulates can be combined, in the appropriate proportions, with a powder blend, granulation or multiparticulates containing the progestin and incorporated into hard gelatin capsules.

This invention also provides a pharmaceutical does pack, containing any number of daily pharmaceutical dosage units. Preferably, and conventionally, the pack contains 28 tablets or multiples thereof. The pack should indicate that the dosage units are to be taken consecutively on a daily basis until the treatment period has ended, or until the pack has been completed. The next pack should be started on the next consecutive day. For combinations containing a unitary dosage tablet containing both conjugated estrogens and MPA, it is preferable that the pack contain one tablet corresponding to each day of administration. For combinations containing separate dosage units of conjugated estrogens and MPA, it is preferable that each one tablet of each correspond to each given day's administration, as indicated on the pill pack.

## Claims

1. Use of conjugated estrogens and medroxyprogesterone acetate in the manufacture of pharmaceutical packs comprising daily dosage units for the treatment or inhibition of menopausal or postmenopausal disorders in a perimenopausal, menopausal, or postmenopausal woman in need thereof, wherein each daily dosage unit comprises a combination of conjugated estrogens and a daily dosage of about 1.5 mg of medroxyprogesterone acetate.

2. The use according to claim 1 which comprises treating or inhibiting vasomotor symptoms in a perimenopausal, menopausal, or postmenopausal woman in need thereof.

3. The use according to claim 2, wherein the vasomotor symptom is hot flushes.

4. The use according to claim 1 which comprises inhibiting or retarding bone demineralization or treating or inhibiting osteoporosis in a perimenopausal, menopausal, or postmenopausal woman in need thereof.

5. The use according to claim 1 which comprises treating or inhibiting vaginal or vulvar atrophy; atrophic vaginitis; vaginal dryness; pruritus; dyspareunia; dysuria; frequent urination; urinary incontinence; urinary tract infections in a perimenopausal, menopausal, or postmenopausal woman in need thereof.

6. The use according to claim 1 which comprises lowering cholesterol, triglycerides, Lp(a), or LDL levels; inhibiting or treating hypercholesteremia; hyperlipidemia; cardiovascular disease; atherosclerosis; peripheral vascular disease; restenosis, vasospasm; or inhibiting vascular wall damage from cellular events leading toward immune mediated vascular damage, in a perimenopausal, menopausal, or postmenopausal woman in need thereof.

7. Use of conjugated estrogens and medroxyprogesterone acetate in the manufacture of pharmaceutical packs comprising daily dosage units for treating or inhibiting free radical involvement in the development of cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, amyotrophic lateral sclerosis, aging effects, adult respiratory distress syndrome, central nervous system trauma and stroke, or injury during reperfusion procedures in a perimenopausal, menopausal, or postmenopausal woman in need thereof, wherein each daily dosage unit comprises a combination of conjugated estrogens and a daily dosage of about 1.5 mg of medroxyprogesterone acetate.

8. Use of conjugated estrogens and medroxyprogesterone acetate in the manufacture of pharmaceutical packs comprising daily dosage units for treating or inhibiting dementias, neurodegenerative disorders, and Alzheimer's disease; providing neuroprotection or cognition enhancement in a perimenopausal, menopausal, or postmenopausal woman in need thereof, wherein each daily dosage unit comprises a combination of conjugated estrogens and a daily dosage of about 1.5 mg of medroxyprogesterone acetate.

9. Use of conjugated estrogens and medroxyprogesterone acetate in the manufacture of pharmaceutical packs comprising daily dosage units for increasing bone mineral density in a perimenopausal, menopausal, or postmenopausal woman in need thereof, wherein each daily dosage unit comprises a combination of conjugated estrogens and a daily dosage of about 1.5 mg of medroxyprogesterone acetate.

10. Use of conjugated estrogens and medroxyprogesterone acetate in the manufacture of pharmaceutical packs comprising daily dosage units of minimizing or reducing levels of breast pain in a woman receiving hormone replacement therapy, wherein each daily dosage unit comprises a combination of conjugated estrogens and a daily dosage of about 1.5 mg of medroxyprogesterone acetate.

11. Use of conjugated estrogens and medroxyprogesterone acetate in the manufacture of pharmaceutical packs comprising daily dosage units for minimizing spotting or breakthrough bleeding, or achieving amenorrhea in a woman receiving hormone replacement therapy, wherein each daily dosage unit comprises a combination of conjugated estrogens and a daily dosage of about 1.5 mg of medroxyprogesterone acetate.

12. The use according to claim 11, wherein the time for minimizing spotting or breakthrough bleeding or achieving the onset of amenorrhea is hastened.

13. The use according to any of claims 1 to 12, wherein the conjugated estrogens is synthetic conjugated estrogens, A.

14. The use according to any of claims 1 to 12, wherein the conjugated estrogens is conjugated equine estrogens, USP.

15. The use according to any of the preceding claims, wherein the daily dosage of conjugated estrogens is between about 0.625 mg and about 0.3 mg.

16. The use according to any of the preceding claims, wherein the daily dosage of conjugated estrogens, is between about 0.45 mg and about 0.3 mg.

17. The use according to any of the preceding claims wherein the daily dosage of conjugated estrogens is about 0.45 mg.

18. The use according to any preceding claim wherein the daily dosage of conjugated estrogens, is about 0.3 mg.

19. The use according to any of the preceding claims wherein the therapeutic product contains fixed daily dosage units.

20. The use according to any of the preceding claims in which the therapeutic product contains at least 28 daily dosage units.

21. The use according to any preceding claim in which the therapeutic product comprises up to 180 daily dosage units.

22. The use according to any of the preceding claims in which the therapeutic product contains at least 30 daily dosage units.

23. The use according to any preceding claim in which the therapeutic product comprises up to 120 daily dosage units.

24. The use according to any preceding claim in which the medroxyprogesterone acetate is micronized.

25. The use according to any preceding claim in which the daily dosage unit is provided once a day.

26. The use according to any preceding claim in which the daily dosage unit is provided for oral administration.

27. The use according to any preceding claim in which the daily dosage unit contains 0.45 mg of conjugated equine estrogens and 1.5 mg of medroxyprogesterone acetate.

28. The use according to any preceding claim in which the daily dosage unit contains 0.3 mg of conjugated equine estrogens and 1.5 mg of medroxyprogesterone acetate.

29. A pharmaceutical composition for use in treating menopausal or postmenopausal disorders in a perimenopausal, menopausal, or postmenopausal woman in need thereof, which comprises conjugated estrogens, a dosage of about 1.5 mg medroxyprogesterone acetate, and a pharmaceutical carrier.

30. A pharmaceutical dosage unit which comprises conjugated estrogens, a dosage of about 1.5 mg of medroxyprogesterone acetate, and a pharmaceutical carrier.

31. A pharmaceutical pack comprising daily dosage units comprising a combination of conjugated estrogens and a daily dosage of about 1.5 mg of medroxyprogesterone acetate

32. The composition, unit or pack according to claim 29 or 30 or 31, wherein the conjugated estrogens is conjugated equine estrogens, USP.

33. The composition, unit or pack according to claim 29 or 30 or 31, wherein the conjugated estrogens is synthetic conjugated estrogens, A.

34. The composition, unit or pack according to claim 32 or 33, wherein the dosage is between about 0.45 mg and about 0.30 mg.

35. The composition, unit or pack according to claim 32 or 33 wherein the dosage is about 0.45 mg or 0.30 mg.

36. The composition, unit or pack according to claim 29 or 30 or 31, wherein the medroxyprogesterone acetate is micronized.

37. The pharmaceutical pack according to claim 31 which comprises up to 180 fixed daily dosage units.

38. The pharmaceutical pack according to claim 37 containing at least 28 fixed daily dosage units.

39. The pharmaceutical pack according to claim 37 containing at least 30 fixed daily dosage units.

## Patentansprüche

1. Verwendung von konjugierten Östrogenen und Medroxyprogesteron Azetat bei der Herstellung pharmazeutischer Packungen, die tägliche Verabreichungsformen zur Behandlung oder Hemmung von menopausalen oder postmenopausalen Beschwerden einer in der perimenopausalen, menopausalen oder postmenopausalen Phase befindlichen Frau, die ihrer bedarf, umfasst, wobei jede tägliche Verabreichungsform eine Kombination konjugierter Östrogene und eine Tagesdosis von etwa 1,5 mg Medroxyprogesteron Azetat umfasst.

2. Verwendung nach Anspruch 1, die die Behandlung oder Hemmung von vasomotorischen Symptomen einer in der perimenopausalen, menopausalen oder postmenopausalen Phase befindlichen Frau, die ihrer bedarf, umfasst.

3. Verwendung nach Anspruch 2, wobei das vasomotorische Symptom Hitzewallungen sind.

4. Verwendung nach Anspruch 1, die die Hemmung oder Verzögerung der Knochendemineralisation oder Behandlung oder Hemmung der Osteoporose einer in der perimenopausalen, menopausalen oder postmenopausalen Phase befindlichen Frau, die ihrer bedarf, umfasst.

5. Verwendung nach Anspruch 1, die die Behandlung oder Hemmung der vaginalen oder vulvären Atrophie, atrophischen Vaginitis, vaginalen Trockenheit, Pruritus, Dyspareunie, Dysurie, häufiges Urinieren, Harninkontinenz, Harntraktinfektionen einer in der perimenopausalen, menopausalen oder postmenopausalen Phase befindlichen Frau, die ihrer bedarf, umfasst.

6. Verwendung nach Anspruch 1, die die Senkung des Cholesterin-, Triglyzeride- und Lp(a)- oder LDL-Spiegels, die Hemmung oder Behandlung der Hypercholesterinämie, Hyperlipidämie, kardiovaskulären Erkrankung, Atherosklerose, peripheren Gefäßerkrankung, eine Restenose, von Vasospasmen oder die Hemmung einer Gefäßwandschädigung durch zelluläre Ereignisse, die zu einem immunvermittelten Gefäßschaden führen, bei einer in der perimenopausalen, menopausalen oder postmenopausalen Phase befindlichen Frau, die ihrer bedarf, umfasst.

7. Verwendung konjugierter Östrogene und Medroxyprogesteron Azetat bei der Herstellung pharmazeutischer Packungen, die tägliche Verabreichungsformen umfassen zur Behandlung oder Hemmung der Beteiligung freier Radikaler an der Entwicklung von Karzinomen, Störungen des Zentralnervensystems, der Alzheimer-Krankheit, von Knochenkrankheiten, Alterungsprozessen, entzündlichen Syndromen, peripheren Gefäßerkrankungen, der rheumatoiden Arthritis, von autoimmunen Krankheiten, des Lungenversagens, Emphysems, zur Vorbeugung gegen einen Reperfusionsschaden, virale Hepatitis, chronische aktive Hepatitis, Tuberkulose, Psoriasis, systemischen Lupus erythematosus, amyotrophische laterale Sklerose, Auswirkungen des Alterns, akutes Lungenversagen bei Erwachsene, Trauma des Zentralnervensystems oder einen Schlaganfall oder Schäden während Reperfusionsverfahren bei einer in der perimenopausalen, menopausalen oder postmenopausalen Phase befindlichen Frau, die ihrer bedarf, wobei jede Tagesverabreichungsform eine Kombination konjugierter Östrogene und eine Tagesdosis von etwa 1,5 mg Medroxyprogesteron Azetat umfasst.

8. Verwendung konjugierter Östrogene und Medroxyprogesteron Azetat bei der Herstellung pharmazeutischer Packungen, die tägliche Verabreichungsformen umfassen zur Behandlung oder Hemmung der Demenz, neurodegenerativer Störungen und Alzheimer-Krankheit und eine Neuroprotektion oder Steigerung des kognitiven Vermögens bei einer in der perimenopausalen, menopausalen oder postmenopausalen Phase befindlichen Frau, die ihrer bedarf, bieten, wobei jede Tagesverabreichungsform eine Kombination konjugierter Östrogene und eine Tagesdosis etwa 1,5 mg Medroxyprogesteron Azetat umfasst.

9. Verwendung konjugierter Östrogene und Medroxyprogesteron Azetat bei der Herstellung pharmazeutischer Packungen, die tägliche Verabreichungsformen umfassen zur Steigerung der knochenmineralischen Dichte bei einer in der perimenopausalen, menopausalen oder postmenopausalen Phase befindlichen Frau, die ihrer bedarf, wobei jede Tagesverabreichungsform eine Kombination konjugierter Östrogene und eine Tagesdosis etwa 1,5 mg Medroxyprogesteron Azetat umfasst.

10. Verwendung konjugierter Östrogene und Medroxyprogesteron Azetat bei der Herstellung pharmazeutischer Packungen, die tägliche Verabreichungsformen umfassen zur Minimierung oder Reduzierung der Schwere der Brustschmerzen bei einer Frau, die mit Hormonersatztherapie behandelt wird, wobei jede Tagesverabreichungsform eine Kombination konjugierter Östrogene und eine Tagesdosis etwa 1,5 mg Medroxyprogesteron Azetat umfasst.

11. Verwendung konjugierter Östrogene und Medroxyprogesteron Azetat bei der Herstellung pharmazeutischer Packungen, die tägliche Verabreichungsformen umfassen zur Minimierung von Schmier- oder Durchbruchblutungen, oder zum Erreichen der Amenorrhoe bei einer Frau, die mit Hormonersatztherapie behandelt wird, wobei jede Tagesverabreichungsform eine Kombination konjugierter Östrogene und eine Tagesdosis etwa 1,5 mg Medroxyprogesteron Azetat umfasst.

12. Verwendung nach Anspruch 11, wobei die Zeit zur Minimierung von Schmier- oder Durchbruchblutungen oder zum Erreichen des Einsetzens der Amenorrhoe beschleunigt ist.

13. Verwendung nach irgendeinem der Ansprüche 1 bis 12, wobei die konjugierten Östrogene synthetische konjugierte Östrogene A sind.

14. Verwendung nach irgendeinem der Ansprüche 1 bis 12, wobei die konjugierten Östrogene konjugierte Stutenöstrogene USP sind.

15. Verwendung nach irgendeinem der vorstehenden Ansprüche, wobei die tägliche Verabreichungsdosis konjugierter Östrogene zwischen etwa 0,625 mg und etwa 0,3 mg beträgt.

16. Verwendung irgendeinem der vorstehenden Ansprüche, wobei die tägliche Verabreichungsdosis konjugierter Östrogene zwischen etwa 0,45 mg und etwa 0,3 mg beträgt.

17. Verwendung nach irgendeinem der vorstehenden Ansprüche, wobei die tägliche Verabreichungsdosis konjugierter Östrogene etwa 0,45 mg beträgt.

18. Verwendung nach irgendeinem der vorstehenden Ansprüche, wobei die tägliche Verabreichungsdosis konjugierter Östrogene etwa 0,3 mg beträgt.

19. Verwendung nach irgendeinem der vorstehenden Ansprüche, wobei das therapeutische Produkt feststehende tägliche Verabreichungsformen enthält.

20. Verwendung nach irgendeinem der vorstehenden Ansprüche, wobei das therapeutische Produkt mindestens 28 tägliche Verabreichungsformen enthält.

21. Verwendung nach irgendeinem der vorstehenden Ansprüche, wobei das therapeutische Produkt bis zu 180 tägliche Verabreichungsformen umfasst.

22. Verwendung nach irgendeinem der vorstehenden Ansprüche, wobei das therapeutische Produkt mindestens 30 tägliche Verabreichungsformen enthält.

23. Verwendung nach irgendeinem der vorstehenden Ansprüche, wobei das therapeutische Produkt bis zu 120 tägliche Verabreichungsformen umfasst.

24. Verwendung nach irgendeinem der vorstehenden Ansprüche, wobei das Medroxyprogesteron Azetat mikronisiert ist.

25. Verwendung nach irgendeinem der vorstehenden Ansprüche, wobei die tägliche Verabreichungsform einmal am Tag verabreicht wird.

26. Verwendung nach irgendeinem der vorstehenden Ansprüche, wobei die tägliche Verabreichungsform zur oralen Verabreichung vorgesehen ist.

27. Verwendung nach irgendeinem der vorstehenden Ansprüche, wobei die tägliche Verabreichungsform 0,45 mg konjugierter Stutenöstrogene und 1,5 mg Medroxyprogesteron Azetat enthält.

28. Verwendung nach irgendeinem der vorstehenden Ansprüche, wobei die tägliche Verabreichungsform 0,3 mg konjugierter Stutenöstrogene und 1,5 mg Medroxyprogesteron Azetat enthält.

29. Pharmazeutische Zusammensetzung zur Behandlung von menopausalen oder postmenopausalen Beschwerden einer in der perimenopausalen, menopausalen oder postmenopausalen Phase befindlichen Frau, die ihrer bedarf, die konjugierte Östrogene, eine Dosierung von etwa 1,5 mg Medroxyprogesteron Azetat und einen pharmazeutischen Träger umfasst.

30. Pharmazeutische Verabreichungsform, die konjugierte Östrogene, eine Dosierung von etwa 1,5 mg Medroxyprogesteron Azetat und einen pharmazeutischen Träger umfasst.

31. Pharmazeutisches Packung, die tägliche Verabreichungsformen umfasst, die eine Kombination von konjugierten Östrogenen und eine tägliche Dosierung von etwa 1,5 mg Medroxyprogesteron Azetat enthalten.

32. Zusammensetzung, Verabreichungsform oder Packung nach Anspruch 29 oder 30 oder 31, wobei die konjugierten Östrogene konjugierte Stutenöstrogene USP sind.

33. Zusammensetzung, Verabreichungsform oder Packung nach Anspruch 29 oder 30 oder 31, wobei die konjugierten Östrogene synthetische konjugierte Östrogene A sind.

34. Zusammensetzung, Verabreichungsform oder Packung nach Anspruch 32 oder 33, wobei die Dosierung zwischen etwa 0,45 mg und etwa 0,30 mg beträgt.

35. Zusammensetzung, Verabreichungsform oder Packung nach Anspruch 32 oder 33, wobei die Dosierung etwa 0,45 mg oder etwa 0,30 mg beträgt.

36. Zusammensetzung, Verabreichungsform oder Packung nach Anspruch 29 oder 30 oder 31, wobei das Medroxyprogesteron Azetat mikronisiert ist.

37. Pharmazeutische Packung nach Anspruch 31, die bis zu 180 feststehende tägliche Verabreichungsformen umfasst.

38. Pharmazeutische Packung nach Anspruch 37, die mindestens 28 feststehende tägliche Verabreichungsformen enthält.

39. Pharmazeutische Packung nach Anspruch 37, die mindestens 30 feststehende tägliche Verabreichungsformen enthält.

## Revendications

1. Utilisation d'oestrogènes conjugués et d'acétate de médroxyprogestérone dans la fabrication de conditionnements pharmaceutiques comprenant des unités posologiques quotidiennes pour le traitement ou l'inhibition de troubles ménopausiques ou post-ménopausiques chez une femme périménopausée, ménopausée, ou post-ménopausée en ayant besoin, dans lesquels chaque unité posologique quotidienne comprend une combinaison d'oestrogènes conjugués et une dose quotidienne d'environ 1,5 mg d'acétate de médroxyprogestérone.

2. Utilisation de la revendication 1, qui comprend le traitement ou l'inhibition des symptômes vasomoteurs chez une femme périménopausée, ménopausée, ou post-ménopausée en ayant besoin.

3. Utilisation selon la revendication 2, dans laquelle le symptôme vasomoteur est les bouffées de chaleur.

4. Utilisation selon la revendication 1, qui comprend l'inhibition ou le retard de la déminéralisation osseuse ou le traitement ou l'inhibition de l'ostéoporose chez une femme périménopausée, ménopausée, ou post-ménopausée en ayant besoin.

5. Utilisation selon la revendication 1, qui comprend le traitement ou l'inhibition de l'atrophie vaginale ou vulvaire ; de la vaginite atrophique ; de la sécheresse vaginale ; du prurit ; de la dyspareunie ; de la dysurie ; de miction fréquente ; de l'incontinence urinaire ; des infections du tractus urinaire chez une femme périménopausée, ménopausée, ou post-ménopausée en ayant besoin.

6. Utilisation selon la revendication 1, qui comprend la réduction des taux de cholestérol, triglycérides, Lp(a), ou LDL ; l'inhibition ou le traitement de l'hypercholestérémie ; de l'hyperlipidémie ; de la maladie cardio-vasculaire ; de l'athérosclérose ; de la maladie vasculaire périphérique ; de la resténose ; du vasospasme ; ou l'inhibition des dégâts de la paroi vasculaire provenant d'événements cellulaires conduisant à des dommages vasculaires à médiation immune, chez une femme périménopausée, ménopausée, ou post-ménopausée en ayant besoin.

7. Utilisation d'oestrogènes conjugués et d'acétate de médroxyprogestérone dans la fabrication de conditionnements pharmaceutiques comprenant des unités posologiques quotidiennes pour le traitement ou l'inhibition de l'implication des radicaux libres dans le développement des cancers, des troubles du système nerveux central, de la maladie d'Alzheimer, des maladies osseuses, du vieillissement, des troubles inflammatoires, de la maladie vasculaire périphérique, de l'arthrite rhumatoïde, des maladies auto-immunes, de la détresse respiratoire, de l'emphysème, de la prévention des lésions de reperfusion, de l'hépatite virale, de l'hépatite active chronique, de la tuberculose, du psoriasis, du lupus érythémateux systémique, de la sclérose latérale amyotrophe, des effets du vieillissement, du syndrome de détresse respiratoire chez l'adulte, du traumatisme du système nerveux central, de l'accident vasculaire cérébral, ou des lésions durant les protocoles de reperfusion chez une femme périménopausée, ménopausée, ou post-ménopausée en ayant besoin, dans lesquels chaque unité posologique quotidienne comprend une association d'oestrogènes conjugués et d'une dose quotidienne d'environ 1,5 mg d'acétate de médroxyprogestérone.

8. Utilisation d'oestrogènes conjugués et d'acétate de médroxyprogestérone dans la fabrication de conditionnements pharmaceutiques comprenant des unités posologiques quotidiennes pour le traitement ou l'inhibition des démences, des maladies neurodégénératives, et de la maladie d'Alzheimer ; la proposition de neuroprotection ou de nootrope chez une femme périménopausée, ménopausée, ou post-ménopausée en ayant besoin, dans lesquels chaque unité posologique quotidienne comprend une combinaison d'oestrogènes conjugués et une dose quotidienne d'environ 1,5 mg d'acétate de médroxyprogestérone.

9. Utilisation d'oestrogènes conjugués et d'acétate de médroxyprogestérone dans la fabrication de conditionnements pharmaceutiques comprenant des unités posologiques quotidiennes pour augmenter la densité minérale osseuse chez une femme périménopausée, ménopausée, ou post-ménopausée en ayant besoin, dans lesquels chaque unité posologique quotidienne comprend une combinaison d'oestrogènes conjugués et une dose quotidienne d'environ 1,5 mg d'acétate de médroxyprogestérone.

10. Utilisation d'oestrogènes conjugués et d'acétate de médroxyprogestérone dans la fabrication de conditionnements pharmaceutiques comprenant des unités posologiques quotidiennes de minimisation ou de réduction des niveaux de douleur au sein chez une femme recevant un traitement hormonal substitutif, dans lesquels chaque unité posologique quotidienne comprend une association d'oestrogènes conjugués et une dose quotidienne d'environ 1,5 mg d'acétate de médroxyprogestérone.

11. Utilisation d'oestrogènes conjugués et d'acétate de médroxyprogestérone dans la fabrication de conditionnements pharmaceutiques comprenant des unités posologiques quotidiennes pour minimiser la microrragie ou la métrorragie ou atteindre l'aménorrhée chez une femme recevant un traitement hormonal substitutif, dans lesquels chaque unité posologique quotidienne comprend une combinaison d'oestrogènes conjugués et une dose quotidienne d'environ 1,5 mg d'acétate de médroxyprogestérone.

12. Utilisation selon la revendication 11, dans laquelle le temps de réduction de la microrragie ou de la métrorragie ou l'atteinte de l'apparition de l'aménorrhée est hâtée.

13. Utilisation selon l'une quelconque des revendications 1 à 12, dans laquelle les oestrogènes conjugués sont des oestrogènes conjugués de synthèse, A.

14. Utilisation selon l'une quelconque des revendications 1 à 12, dans laquelle les oestrogènes conjugués sont des oestrogènes conjugués équins, USP.

15. Utilisation selon l'une quelconque des revendications précédentes, dans laquelle la dose quotidienne d'oestrogènes conjugués se situe entre environ 0,625 mg et environ 0,3 mg.

16. Utilisation selon l'une quelconque des revendications précédentes, dans laquelle la dose quotidienne d'oestrogènes conjugués se situe entre environ 0,45 mg et environ 0,3 mg.

17. Utilisation selon l'une quelconque des revendications précédentes, dans laquelle la dose quotidienne d'oestrogènes conjugués est d'environ 0,45 mg.

18. Utilisation selon l'une quelconque des revendications précédentes, dans laquelle la dose quotidienne d'oestrogènes conjugués est d'environ 0,3 mg.

19. Utilisation selon l'une quelconque des revendications précédentes, dans laquelle le produit thérapeutique contient des unités posologiques quotidiennes fixes.

20. Utilisation selon l'une quelconque des revendications précédentes, dans laquelle le produit thérapeutique contient au moins 28 unités posologiques quotidiennes.

21. Utilisation selon l'une quelconque des revendications précédentes, dans laquelle le produit thérapeutique comprend jusqu'à 180 unités posologiques quotidiennes.

22. Utilisation selon l'une quelconque des revendications précédentes, dans laquelle le produit thérapeutique contient au moins 30 unités posologiques quotidiennes.

23. Utilisation selon l'une quelconque des revendications précédentes, dans laquelle le produit thérapeutique contient jusqu'à 120 unités posologiques quotidiennes.

24. Utilisation selon l'une quelconque des revendications précédentes, dans laquelle l'acétate de médroxyprogestérone est micronisé.

25. Utilisation selon l'une quelconque des revendications précédentes, dans laquelle l'unité posologique quotidienne est fournie une fois par jour.

26. Utilisation selon l'une quelconque des revendications précédentes, dans laquelle l'unité posologique quotidienne est fournie par administration orale.

27. Utilisation selon l'une quelconque des revendications précédentes, dans laquelle l'unité posologique quotidienne contient 0,45 mg d'cestrogènes conjugués équins et 1,5 mg d'acétate de médroxyprogestérone.

28. Utilisation selon l'une quelconque des revendications précédentes, dans laquelle l'unité posologique quotidienne contient 0,3 mg d'oestrogènes conjugués équins et 1,5 mg d'acétate de médroxyprogestérone.

29. Composition pharmaceutique pour l'utilisation dans le traitement des troubles de la ménopause ou de la post-ménopause chez une femme périménopausée, ménopausée, ou post-ménopausée en ayant besoin, qui comprend des oestrogènes conjugués, une dose d'environ 1,5 mg d'acétate de médroxyprogestérone, et un support pharmaceutique.

30. Unité posologique pharmaceutique qui comprend des oestrogènes conjugués, une dose d'environ 1,5 mg d'acétate de médroxyprogestérone, et un support pharmaceutique.

31. Conditionnement pharmaceutique comprenant des unités posologiques quotidiennes comprenant une combinaison d'oestrogènes conjugués et une dose quotidienne d'environ 1,5 mg d'acétate de médroxyprogestérone.

32. Composition, unité ou conditionnement selon la revendication 29 ou 30 ou 31, dans laquelle(lequel) les oestrogènes conjugués sont des oestrogènes conjugués équins, USP.

33. Composition, unité ou conditionnement selon la revendication 29 ou 30 ou 31, dans laquelle(lequel) les oestrogènes conjugués sont des oestrogènes conjugués de synthèse, A.

34. Composition, unité ou conditionnement selon la revendication 32 ou 33, dans laquelle(lequel) la dose si situe entre environ 0,45 mg et environ 0,30 mg.

35. Composition, unité ou conditionnement selon la revendication 32 ou 33, dans laquelle(lequel) la dose est d'environ 0,45 mg ou 0,30 mg.

36. Composition, unité ou conditionnement selon la revendication 29 ou 30 ou 31, dans laquelle(lequel) l'acétate de médroxyprogestérone est micronisé.

37. Conditionnement pharmaceutique selon la revendication 31, qui comprend jusqu'à 180 unités posologiques quotidiennes fixes.

38. Conditionnement pharmaceutique selon la revendication 37, qui contient au moins 28 unités posologiques quotidiennes fixes.

39. Conditionnement pharmaceutique selon la revendication 37, contenant au moins 30 unités posologiques quotidiennes fixes.
